(19) Europäisches Patentamt
European Patent Office
Office européen des brevets

(11) **EP 4 349 964 A1**

(12) **EUROPEAN PATENT APPLICATION**
published in accordance with Art. 153(4) EPC

(43) Date of publication:
**10.04.2024  Bulletin 2024/15**

(21) Application number: **22816387.9**

(22) Date of filing: **26.05.2022**

(51) International Patent Classification (IPC):
*C12N 5/071* (2010.01)    *C12N 5/0775* (2010.01)
*C12N 5/077* (2010.01)    *A61K 35/28* (2015.01)
*A61K 48/00* (2006.01)    *A61P 17/02* (2006.01)

(52) Cooperative Patent Classification (CPC):
**A61K 35/28; A61K 48/00; A61P 17/02; C12N 5/06**

(86) International application number:
**PCT/KR2022/007486**

(87) International publication number:
**WO 2022/255728 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2021  KR 20210070055**
**25.05.2022  KR 20220064290**

(71) Applicant: **Seoul National University Hospital Seoul 03080 (KR)**

(72) Inventors:
• **KWON, Seong Keun**
  **Seoul 06004 (KR)**
• **CHOI, Ji Suk**
  **Seoul 02583 (KR)**
• **EOM, Min Rye**
  **Seoul 01173 (KR)**
• **JEONG, Eun Ji**
  **Seoul 08026 (KR)**

(74) Representative: **Nederlandsch Octrooibureau P.O. Box 29720 2502 LS The Hague (NL)**

(54) **PREPARATION AND STANDARDIZATION OF CELL SPHEROIDS FOR PROMOTING TISSUE REGENERATION**

(57)    A standardization method for preparing cell spheroids according to the present invention provides a standardization method for preparing cell spheroids with remarkably increased tissue regenerative capacity, and enables the mass production of cell spheroids which can be directly applied to treatments, and also enables the mass production of therapeutic cell spheroids which have significantly increased tissue regenerative capacity, that is, stable quality, thus enabling industrialization of cell spheroids, and making it possible to effectively apply cell spheroids to the treatment of various diseases.

**FIG 3**

EP 4 349 964 A1

## Description

[Technical Field]

The present invention relates to a method for producing and standardizing a cell spheroid for promoting tissue regeneration, and particularly to a method for producing, selecting, and quality controlling the cell spheroid, which is the most effective for treatment, by standardizing a method for producing a spheroid for a cell therapy agent, which can be applied to the treatment of diseases requiring tissue regeneration, and the like.

[Background Art]

Stem cells are cells that can differentiate into various cells that constitute biological tissues, and collectively refer to undifferentiated cells in the pre-differentiation stage, which can be obtained from each tissue of an embryo, a fetus and an adult. Stem cells are characterized by having properties of being able to differentiate into specific cells according to the differentiation stimulus (environment) and producing cells that are the same as themselves through cell division (self-renewal), and having a flexibility (plasticity) with which the stem cells can differentiate into different cells according to the differentiation stimulus. Stem cells can be divided into pluripotent, multipotent, and unipotent stem cells according to their differentiation ability. Pluripotent stem cells are cells with pluripotency, which have the potential to differentiate into all types of cells, and some stem cells have pluripotent or unipotent potential. A lot of research and development has been actively conducted with regard to the application of such differentiation ability of stem cells to a cell therapy agent. In addition to stem cells, a lot of research has been conducted even for application to a cell therapy agent for restoring the function of cells and tissues through a series of actions such as proliferating and selecting autologous, allogenic, and xenogeneic somatic cells *ex vivo*, or changing the biological characteristics of cells by various methods (Segers, Vincent FM, and Richard T. Lee. Nature (2008) 451(7181): 937-942).

Many possibilities have been proposed for restoring the functions of cells and tissues by applying cell therapeutic agents to various diseases that require the recovery and regeneration of lost cells such as neurological diseases, heart diseases, lung diseases, liver diseases, and cancer resection through various studies. However, there is a lack of research into standardization methods for a cell therapy agent capable of securing their therapeutic effects when directly applied clinically.

Thus, as a result of intensive studies to develop a standardization method for actually using a cell therapy agent for treatment, the present inventors have completed a method for standardizing a cell spheroid, which remarkably increases the therapeutic effect of the present invention. The cell spheroid according to the present invention is expected to rationally derive the administration dose according to the type of disease and the status of a patient by making it possible to standardize the pharmacological activity of a cell therapy agent, and to contribute to the development of the cell therapy agent by remarkably increasing the therapeutic efficiency in the clinical setting to make the cell therapy agent actually applicable to the clinical setting.

[Disclosure]

[Technical Problem]

The present invention has been devised to solve the above-described problems in the related art, and an object thereof is to provide a method for producing a cell spheroid with remarkably increased tissue regeneration capacity, the method including: a) measuring the diameter of cells; b) seeding $1 \times 10^3$ to $1 \times 10^{10}$ cells into a culture dish when the diameter of cells is 1 to 50 $\mu$m; and (c) obtaining a cell spheroid by culturing the cells, a cell spheroid produced using the same, and the like.

However, the technical problems which the present invention intends to solve are not limited to the technical problems, which have been mentioned above, and other technical problems, which have not been mentioned, will clearly be understood by those with ordinary skill in the art to which the present invention pertains from the following description.

[Technical Solution]

The present invention provides a method for producing a cell spheroid, the method including: a) measuring the diameter of cells; b) seeding preferably $1 \times 10^3$ to $1 \times 10^{10}$, more preferably $3 \times 10^3$ to $3 \times 10^6$ cells into a culture dish when the diameter of cells is preferably 1 to 50 $\mu$m, more preferably 3 to 30 $\mu$m; and c) obtaining a cell spheroid by culturing the cells.

In an exemplary embodiment of the present invention, in step b), it is preferred that cells are seeded into a culture dish in the initial required number of single cells (f), calculated by the following equation.

$$f = a \cdot exp^{-0.5\left[\left(\frac{x-x_0}{b}\right)^2 + \left(\frac{y-y_0}{c}\right)^2\right]}$$

In the above equation, f denotes the initial required number of single cells, x denotes the diameter of a single cell, and y denotes the diameter of a cell spheroid, and preferably $x_0$ is 8.2775 to 10.3231, $y_0$ is 624.8576 to 726.3632, a = 53.0017 to 89.4827, b = 9.0232 to 10.2778, and c = 162.0563 to 191.5685, and more preferably, $x_0$ = 7 to 11, $y_0$ = 650 to 700, a = 50 to 90, b = 7 to 11, and c = 150 to 200, but these variables are not limited thereto.

In another exemplary embodiment of the present invention, in step c), the cells may be cultured for preferably 2 to 7 days, more preferably 2 to 4 days.

In still another exemplary embodiment of the present invention, the cell spheroid may have a diameter of preferably 100 to 500 $\mu$m, more preferably 200 to 400 $\mu$m, and even more preferably 200 to 300 $\mu$m.

In yet another exemplary embodiment of the present invention, for the cells, somatic cells, stem cells or a mixture thereof, that is, a mixture of somatic cells and stem cells, may be used, the stem cells may be embryonic stem cells, adult stem cells, induced pluripotent stem cells, mesenchymal stem cells derived therefrom, or the like, the embryonic stem cells may be mesenchymal stem cells derived from embryonic stem cells, the adult stem cells may be stem cells derived from various tissues such as bone marrow, fat, the liver, teeth, dental pulp, the brain, the heart, the kidneys, skin, hair follicles, the intestines, the lungs, lymph nodes, muscles, bones, and salivary glands, but are not limited thereto as long as they are undifferentiated cells with the ability to differentiate into various types of body tissue cells. The somatic cells are fibroblasts, salivary gland cells, muscle cells, airway epithelial cells, kidney cells, liver cells, heart cells, brain cells, lung cells, bone cells, esophageal epithelial cells, gastrointestinal cells, mucosal cells, cartilage cells, thyroid cells, adipocytes, taste cells, skin cells, corneal endothelial cells, nerve cells, thymocytes, internal auditory canal cells, nasal cavity cells, hair root cells, and the like, but are not limited thereto as long as they are cells that make up animals and plants.

In yet another exemplary embodiment of the present invention, the cell spheroid is characterized in that by inducing a hypoxic environment therein, the secretion of various pharmacologically active factors such as angiogenic factors and cell growth factors is promoted, thereby effectively promoting tissue regeneration.

Further, the present invention provides a cell spheroid produced by the method.

In addition, the present invention provides a cell therapy agent for promoting tissue regeneration, which includes the cell spheroid produced by the method as an active ingredient.

In an exemplary embodiment of the present invention, the cell therapy agent may include preferably 10 to 500 cell spheroids, but the amount is not limited thereto as long as it can promote tissue regeneration *in vivo.*

Furthermore, the present invention provides a tissue regeneration method including administering to a subject the cell spheroid produced by the method.

Further, the present invention provides a use of the cell spheroid produced by the method for tissue regeneration.

[Advantageous Effects]

The method for producing a cell spheroid according to the present invention provides a standardization method for producing a cell spheroid with remarkably increased tissue regeneration capacity, thereby making it possible to industrialize and produce a cell spheroid that can be directly applied to treatment. More specifically, by quantifying the initial required number of single stem cells using the function between the diameter of a cell and the diameter of a cell spheroid with remarkably increased tissue regeneration capacity, it is expected that it will be possible to mass-produce a therapeutic cell spheroid with significantly increased tissue regeneration capacity, that is, stable quality. Therefore, it is expected that it is possible to effectively apply the method for producing a cell spheroid of the present invention to the treatment through cell therapy of various diseases that can be treated through a mechanism such as angiogenesis, cell regeneration, and tissue regeneration using the method for producing a cell spheroid of the present invention.

[Description of Drawings]

FIG. 1 is a set of views showing the results of confirming the diameter of a cell spheroid according to the number of cells according to an exemplary embodiment of the present invention.

FIG. 2 is a set of views showing the results of a logistic regression analysis of the correlation among the diameter of a single cell, the diameter of a cell spheroid, and the initial number of single cells according to an exemplary embodiment of the present invention. The blue dotted line in FIG. 2 indicates the regression line.

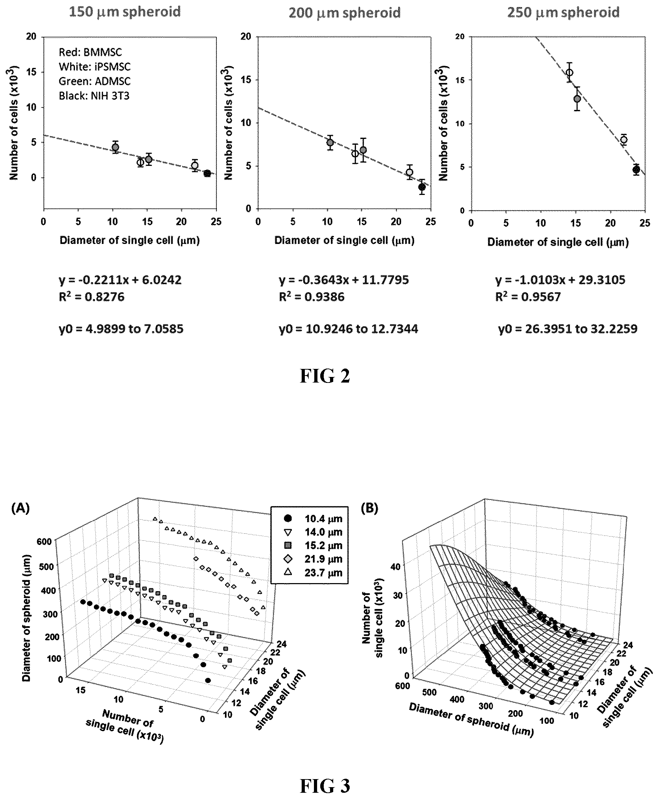

FIG. 3 shows the results of showing the correlation among the diameter of a single cell, the diameter of a cell spheroid, and the initial number of single cells according to an exemplary embodiment of the present invention by 3D graphs. (A) shows the diameter of the cell spheroid according to the diameter of a single cell and the number of single cells, where the black dot denotes a single cell with a diameter of 10.4 $\mu$m, the white dot denotes a single cell with a diameter of 14.0 $\mu$m, the red dot denotes a single cell with a diameter of 15.2 $\mu$m, the green dot denotes a single cell with a diameter of 21.9 $\mu$m, and the yellow dot denotes a single cell with a diameter of 23.7 $\mu$m. (B) shows the diameter (x) of a single cell and the diameter (y) of a spheroid to predict the number of single cells (f) required for the preparation of a cell spheroid, where the blue mesh denotes a regression (3D, Gaussian).

FIG. 4 is a set of views showing the results of applying a function for calculating the required number of single cells in various culture dishes according to an exemplary embodiment of the present invention.

FIG. 5 is a set of views showing the results of observing the degree of hypoxia inside the spheroid according to the diameter of the cell spheroid according to an embodiment of the present invention under a fluorescence microscope.

FIG. 6 is a view showing the results of confirming cell viability according to the diameter of the cell spheroid according to an exemplary embodiment of the present invention.

FIG. 7 is a set of views showing the results of confirming the accumulative secretion amount of pharmacologically active factors according to the diameter of the cell spheroid according to an exemplary embodiment of the present invention using ELISA.

FIG. 8 is a view schematically showing an experimental method (A) for confirming the angiogenesis-promoting effect of a cell spheroid and a method for interpreting the corresponding results (B) according to an exemplary embodiment of the present invention.

FIG. 9 is a view showing the results of confirming the angiogenesis-promoting effects of the cell spheroid according to an exemplary embodiment of the present invention.

FIG. 10 is a set of views showing the results of quantifying the angiogenesis-promoting effect of a cell spheroid according to an exemplary embodiment of the present invention using Imaged.

FIG. 11 is a set of views showing the results of confirming the effect of the cell spheroid according to an exemplary embodiment of the present invention on reconstructing aging vocal folds.

FIG. 12 is a set of views showing an appearance in which a cell spheroid according to an exemplary embodiment of the present invention is transplanted into a defective organ. Black arrows indicate a cell spheroid applied to a nanofiber membrane.

FIG. 13 is a view showing the results of endoscopically confirming the tissue restoration effect of the cell spheroid according to an exemplary embodiment of the present invention. * indicates a PCL nanofiber membrane.


[Modes of the Invention]

As a result of intensive studies on standardization methods essential for commercializing cell therapy agents, the present inventors confirmed that cell spheroids with a diameter of 200 to 400 $\mu$m may induce a hypoxic environment therein to promote the secretion of various pharmacologically active factors, thereby remarkably increasing the tissue regeneration capacity of the cell spheroid. In addition, by inventing a function capable of quantifying the initial required number of cells to prepare a cell spheroid with a diameter of 200 to 400 $\mu$m using the cell diameter, the cell spheroid and the preparation method thereof were standardized. Therefore, it is expected that it will be possible to commercialize and mass-produce a cell spheroid with remarkably increased tissue regeneration capacity, that is, with remarkably increased therapeutic effects using the method for producing cell spheroids of the present invention.

As used herein, the "stem cell" refers to a general concept of differentiated cells having the ability to differentiate into various types of body tissue cells and self-renewal, that is, undifferentiated cells having sternness. These stem cells are divided into embryonic stem cells, adult stem cells, induced pluripotent stem cells, mesenchymal stem cells derived therefrom, or the like, embryonic stem cells refer to the cell mass stage before forming specific organs within 14 days after fertilization, and also include mesenchymal stem cells produced using the embryonic stem cells, and recently, induced pluripotent derived mesenchymal stem cells (iPS-MSCs) have also been produced from normal cells through dedifferentiation. Therefore, the stem cells are not limited thereto as long as the stem cells are cells capable of differentiating into all cells and tissues constituting the body. Adult stem cells are extracted from various tissues such as umbilical cord blood, bone marrow, blood, bones, cartilage, the brain, hair follicles, dental pulp, teeth, the heart, and salivary glands, and refer to primitive cells immediately before differentiation into cells of specific organs, but are not limited thereto.

As used herein, somatic cells refer to cells that make up the adult body with limited differentiation and self-renewal abilities, and may specifically be fibroblasts, salivary gland cells, muscle cells, airway epithelial cells, kidney cells, liver cells, heart cells, brain cells, lung cells, osteocytes, esophageal epithelial cells, gastrointestinal cells, mucosal cells, cartilage cells, thyroid cells, adipocytes, taste cells, skin cells, corneal endothelial membrane, nerve cells, thymocytes, internal auditory canal cells, nasal cavity cells, hair root cells, and the like. Further, the somatic cells may be

EP 4 349 964 A1

fibroblasts in the embryonic period, or may be naturally occurring somatic cells or genetically modified somatic cells. However, the somatic cells are not limited thereto as long as they are derived from plants or animals and have limited differentiation and self-renewal abilities.

As used herein, "culture medium" refers to a liquid or solid material in which nutrients, osmotic pressure, pH, and the like are adjusted to a level required for culturing, according to the purpose such as proliferation, preservation, and transportation of cells, tissues, or bacteria, and preferably includes all media typically used for culturing cells, and for example, a basal medium such as Dulbecco's Modified Eagle's Medium (DMEM), a minimal essential medium (MEM), Basal Medium Eagle (BME), RPMI1640, F-10, F-12, Glasgow's Minimal Essential Medium (GMEM), and Iscove's Modified Dulbecco's Medium (IMDM) or a mixture thereof may be used, but the culture medium is not limited thereto as long as it is a medium generally used to culture cells.

As used herein, "diameter" refers to the diameter of a cell or cell spheroid, and to the length of the longest straight line passing through the center.

In the present specification, a "culture dish" is a general container used to culture cells, and may also be a large culture container used in factories for mass production, and particularly, includes a culture dish for producing a cell spheroid. The culture dish for producing a cell spheroid may include a gel-like mold with a groove of a certain size, a container for suppressing cell adhesion, and the like, the gel-type mold may be a mold made of agar, alginate, gellan gum, hypromellose (hydroxypropyl methylcellulose), Pluronic, poly(N-isopropylacrylamide) (PNIPAM), methacrylic acid, or polymers of these series, and the container for suppressing cell adhesion has extremely high hydrophilicity or hydrophobicity, which means that the adsorption of materials involved in cell adhesion is suppressed, thereby suppressing the phenomenon of cells adhering to the culture dish during cell culture, may be a hydroxyethyl methacrylate (HEMA) polymer, polydimethylsiloxane (PDMS), a 2-methacryloyloxyethyl phosphorylcholine polymer, lipidure coat (AMSBIO), Nunc HydroCell Surface, Corning Ultra-Low Attachment Surface, and the like, and may be a bottomless culture container with a groove of a certain size using a hanging drop method, but is not limited thereto as long as it is a culture dish used to prepare a stem cell spheroid.

As used herein, a "cell spheroid" refers to a cell cultured as a three-dimensional spherical body, and since the morphology of cells in the body is originally three-dimensional, cell growth in the form of a spheroid is excellent and the original characteristics of the cells may be well maintained compared to cells cultured in a monolayer. Furthermore, the cell spheroid refers to a three-dimensional cell aggregate in which the expression of factors exhibiting various pharmacological activities is increased as a hypoxic environment is induced by limiting the transfer of oxygen to the center of the cell spheroid. For the cell spheroid of the present invention, it is known that the secretion of cytokines required for the growth and differentiation of cells, such as a vascular epithelial growth factor (VEGF), matrix metalloproteinases (MMPs), and a basic fibroblast growth factor (bFGF), epidermal growth factor (EGF), and immune-related cytokines, such as the interleukin family, which are factors that exhibit the pharmacological activity, that is, tissue regeneration promoting factors known to promote tissue regeneration, is increased, and in particular, VEGF promotes tissue regeneration by promoting angiogenesis to recruit cytokines and immune cells required for tissue regeneration to damaged tissues. Therefore, the cell spheroid of the present invention in which the expression of such factors is promoted may be effectively used for the treatment of various diseases requiring tissue regeneration. Various diseases that require tissue regeneration are a general term for all diseases that require tissue regeneration due to aging, damage, necrosis, and the like, may include tissues such as, for example, bones, cartilage, skin, eardrum, the lungs, fat, cartilage, bones, nerves, ligaments, tendons, vocal folds, the heart, the liver, the uterus, salivary glands, the trachea, and the esophagus, or the cell spheroid may also be used to regenerate tissue damaged by ischemic diseases, and the ischemic diseases may include cardiac angina, myocardial infarction, cerebral infarction, foot ulcers, and the like. Alternatively, the cell spheroid may also be used for tissue regeneration after tissue resection surgery such as cancer resection surgery. However, the disease is not limited thereto, as long as it can be treated by a method of regenerating tissue using stem cells.

As used herein, the term "cell therapy agent" refers to a drug used for the purpose of treatment, diagnosis, and prevention as a cell spheroid using somatic cells or stem cells produced through isolation from a mammal, culture and specific manipulation (US FDA regulations), and specifically, it refers to a drug used for the purpose of treatment, diagnosis, and prevention through a series of actions of *in vitro* proliferating and selecting allogenic and xenogenic cells or changing the biological characteristics of cells by other methods for the purpose of recovering the functions of cells or tissues. The cell therapy agent of the present invention may contain one or more active ingredients that exhibit the same or similar functions in addition to the cell spheroid. In addition, the cell therapy agent may be produced by further including one or more pharmaceutically acceptable carriers for administration. As the pharmaceutically acceptable carrier, a binder, a lubricant, a disintegrant, an excipient, a solubilizing agent, a dispersing agent, a stabilizer, a suspending agent, a colorant, a flavoring agent, and the like may be used when orally administered, in the case of injection, a buffering agent, a preservative, an analgesic, a solubilizer, an isotonic agent, a stabilizer, and the like may be mixed and used, and in the case of topical administration, a base, an excipient, lubricant, a preservative, and the like may be used. The formulation of the cell therapy agent of the present invention may be variously produced by mixing the pharmaceutical composition of the present invention with the pharmaceutically acceptable carrier as described above.

5

For example, the formulation may be produced in the form of a tablet, a troche, a capsule, an elixir, a suspension, a syrup, a wafer, and the like for oral administration, and in the case of injection, the injection may be formulated into unit dosage ampoules or in multiple dosage forms. The pharmaceutical composition of the present invention may be formulated into other solutions, suspensions, tablets, capsules, sustained-release preparations, and the like.

The route of administration of the cell therapy agent according to the present invention includes, but is not limited to, oral, intravenous, intramuscular, intraarterial, intramedullary, intrathecal, intracardiac, transdermal, subcutaneous, intraperitoneal, intranasal, intestinal, topical, sublingual or rectal routes. Oral or parenteral administration is preferred. As used herein, the term "parenteral" includes subcutaneous, intradermal, intravenous, intramuscular, intraarticular, intrasynovial, intrasternal, intrathecal, intralesional administration and intracranial injection or infusion techniques.

The stem cell therapy agent of the present invention may vary widely according to various factors including the activity of the cell therapy agent used, age, body weight, general health, sex, dosage, administration time, administration route, excretion rate, drug combination, and severity of the specific disease to be prevented or treated, and may be appropriately selected by those skilled in the art. For example, the cell spheroid may be administered in a dose of 10 to 500 spheroids, preferably 15 to 300 spheroids. The cell spheroid may also be administered once or in several divided doses a day. Furthermore, when the cell spheroid is formulated as a liquid unit preparation such as a solution, a suspension, or an emulsion, it may also be administered to a patient at the above cell concentration.

As used herein, "subject" refers to a subject to which the composition of the present invention may be administered, and the subject is not limited.

Hereinafter, preferred examples for helping the understanding of the present invention will be suggested. However, the following examples are provided only so that the present invention may be more easily understood, and the content of the present invention is not limited by the following examples.

### [Examples]

### Example 1. Preparation of cell spheroid

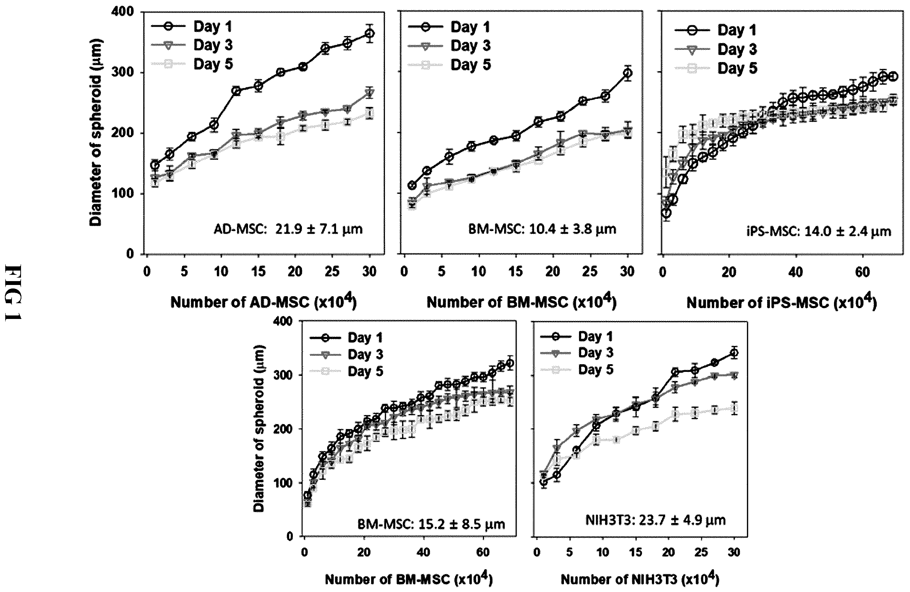

In order to prepare a cell spheroid, first, bone marrow-derived mesenchymal stem cells (BM-MSCs), adipose-derived mesenchymal stem cells (AD-MSCs), induced pluripotent-derived mesenchymal stem cells (iPS-MSCs), and NIH 3T3 fibroblasts cultured in a monolayer were each treated with trypsin/EDTA and isolated into single cells. Then, the isolated single cells were prepared at various concentrations, and seeded at 50 $\mu$L each together with a culture medium in an agar mold with 35 grooves of a certain size, and cultured under the conditions of 37°C and 5% $CO_2$. The initial number of cells used for culture was $1 \times 10^4$ to $30 \times 10^4$ cells/well for AD-MSCs and NIH3T3 fibroblasts, and $1 \times 10^4$ to $69 \times 10^4$ cells/well for BM-MSCs and iPS-MSCs. Then, the diameter of the cell spheroids according to the number of single cells was measured on days 1, 3, and 5. The results are shown in FIG. 1.

As shown in FIG. 1, it was confirmed that 35 cell spheroids were produced, and the diameter of the cell spheroids on day 1 of culture was larger than that on days 3 and 5, whereas as the culture time elapsed, i.e., on days 3 and 5, the diameter decreased. Through this, it can be confirmed that as time passed, the diameter of cell spheroids was decreased as reactions such as cell-cell interactions and cell-ECM interactions progressed, and that changes in diameter were noticeable in the early stage of spheroid formation, and thereafter the amount was insignificant.

### Example 2: Determination of required number of single cells according to diameter of cell spheroids

To determine the required number of single cells according to the diameter of the cell spheroids, the diameter of the cell spheroids formed after 3 days was measured, the relationship between initial single cell number and cell diameter for each cell spheroid with a diameter of 150 $\pm$ 10 $\mu$m, 200 $\pm$ 10 $\mu$m, and 250 $\pm$ 10 $\mu$m was analyzed by a logistic regression (Polynominal, Linear regression, SigmaPlot12.0), and a regression line was drawn. The diameters of single cells were confirmed to be 10.4 $\pm$ 3.8 $\mu$m and 15.2 $\pm$ 8.5 $\mu$m for BM-MSCs, 14.0 $\pm$ 2.4 $\mu$m for iPS-MSCs, 21.9 $\pm$ 7.1 $\mu$m for AD-MSCs, and 23.7 $\pm$ 4.9 $\mu$m for NIH3T3. The results are shown in FIG. 2.

As shown in FIG. 2, it was confirmed through the regression line that each single cell diameter can be used to calculate the number of single cells required to prepare a cell spheroid with a target diameter.

Then, the results were expressed as a 3D graph using a rotation matrix, and the correlation of the number of single cells with the diameter of the single cell and the diameter of the cell spheroid was expressed as a function. The results are shown in FIG. 3.

As shown in FIG. 3, the function to determine the initial required number of single cells required to prepare a cellular spheroid with a target diameter is as follows.

$$f = a \cdot exp^{-0.5\left[\left(\frac{x-x_0}{b}\right)^2 + \left(\frac{y-y_0}{c}\right)^2\right]}$$

It was confirmed that in the above equation, f denotes the initial required number of single cells, x denotes the diameter of a single cell, and y denotes the diameter of a cell spheroid, a significant result is exhibited when $x_0$ is 8.2775 to 10.3231, $y_0$ is 624.5876 to 726.3632, a = 53.0017 to 89.4827, b = 9.0232 to 10.2778, and c = 162.0563 to 191.5685, and in particular, the equation shows $R^2$ = 0.9631 when $x_0$ = 9.3003, $y_0$ = 675.4754, a = 71.2422, b = 9.6505, and c = 176.8124. Therefore, it was confirmed through the function that the initial required number of single cells for producing a cell spheroid with a desired diameter can be quantified.

**Example 3: Confirmation of method of measuring required number of single cells according to cell spheroid diameter**

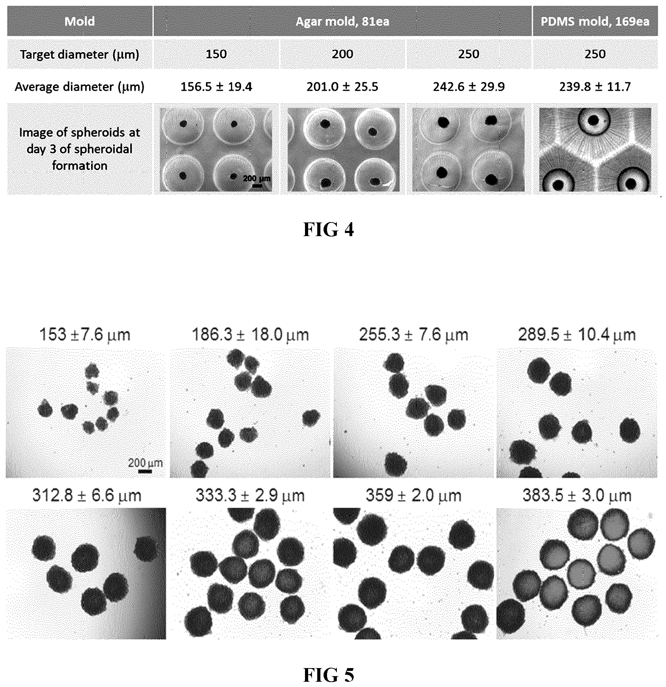

In order to confirm whether the method of measuring the required number of single cells of the present invention can be applied to various culture dishes, experiments were conducted using culture dishes having various sizes and shapes. More specifically, it was confirmed whether a cell spheroid with a target diameter was produced by seeding AD-MSC single cells into an agar mold with 81 circular grooves of a certain size or a PDMS mold with 169 hexagonal grooves in the number required to produce a spheroid with a desired diameter, and culturing the cells for 3 days. The results are shown in FIG. 4.

As shown in FIG. 4, it was confirmed that cell spheroids with the desired diameter were produced regardless of the type of culture dish. Through the above results, it could be confirmed that the function for calculating the initial required number of single cells of the present invention can be applied regardless of the size and morphology of the culture dish.

**Example 4. Confirmation of characteristics of cell spheroids**

**4.1. Confirmation of hypoxic environment induction according to diameter of cell spheroid**

In order to confirm the degree of hypoxic environment induction inside the spheroid according to the diameter of the cell spheroid, the spheroid was treated with lectin-like oxidized low-density lipoprotein (LDL) receptor-1 (Lox-1) used to confirm the hypoxia condition in AD-MSC spheroids on day 1 and further cultured for 1 day. Then, the degree of hypoxia was confirmed using a fluorescence microscope. The results are shown in FIG. 5.

As shown in FIG. 5, it was confirmed that no hypoxic conditions were formed inside the cell spheroid with a diameter of 186 $\mu$m through the fact that no red fluorescence was observed, but red fluorescence was observed in the cell spheroid with a diameter of 200 $\mu$m or more. Through the above results, it could be confirmed that when the diameter of the cell spheroid is 200 $\mu$m or more, a hypoxic environment is induced inside, and through this, it could be confirmed that the expression level of bioactive factors expressed in the hypoxic environment was increased.

**4.2. Confirmation of cell viability according to diameter of cell spheroid**

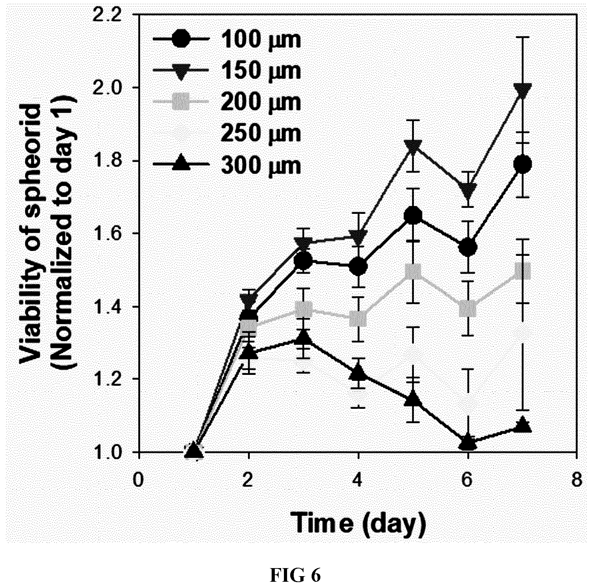

In order to confirm cell viability according to the diameter of the cell spheroid, stem cell spheroids with a diameter of 100 to 300 $\mu$m were produced using mesenchymal stem cells derived from embryonic cells using a function for obtaining the initial required number of single cells required to produce a cell spheroid with a target diameter established in Example 2, and then further cultured for 7 days therefrom, and cell viability was confirmed using Alamar Blue assay. The results are shown in FIG. 6.

As shown in FIG. 6, it could be confirmed that cell viability was reduced in cell spheroids with a diameter of 250 $\mu$m or more, and a hypoxic environment inside the spheroid was induced, thereby increasing cell death.

**4.3. Confirmation of degree of secretion of pharmacologically active factors according to diameter of cell spheroid**

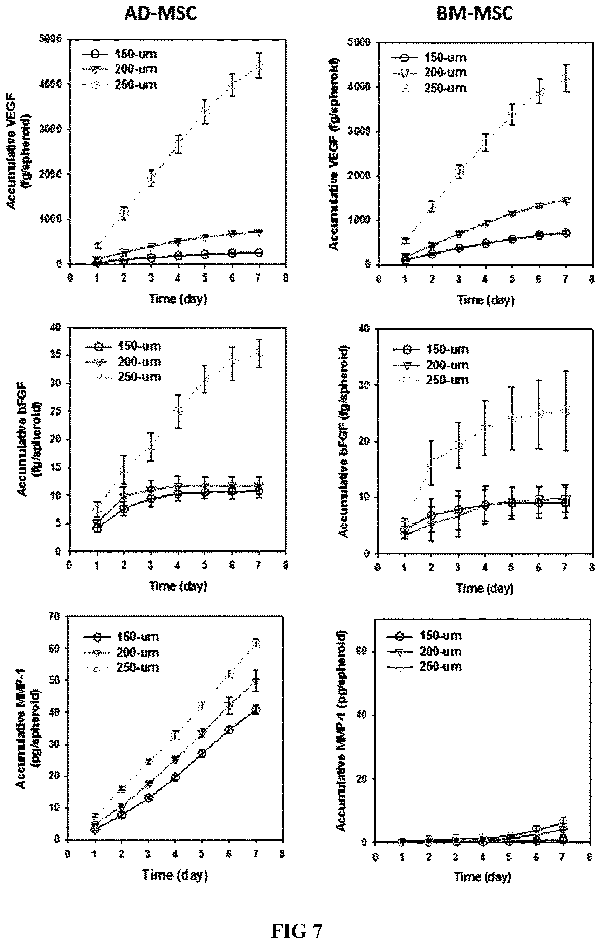

In order to confirm the degree of secretion of pharmacologically active factors according to the diameter of the cell spheroid, each single cell (50 $\mu$L) was dispensed into an agar mold with 35 grooves of a certain size, and then cultured under the conditions of 37°C and 5% $CO_2$ after additionally dispensing 450 $\mu$L of a culture medium. For the initial number of cells used for culture, $1.71 \times 10^3$ cells for AD-MSCs and $2.57 \times 10^3$ cells for BM-MSCs were each dispensed in order for the cell spheroid to have a diameter of 150 $\mu$m on day 3 of culture per one spheroid, $4.28 \times 10^3$ cells for AD-MSCs and $6.00 \times 10^3$ cells for BM-MSCs were each dispensed in order for the cell spheroid to have a diameter of 200 $\mu$m, and $8.57 \times 10^3$ cells for AD-MSCs and $12.86 \times 10^3$ cells for BM-MSCs were each dispensed in order to the cell spheroid to have a diameter of 250 $\mu$m. Then, the culture medium was replaced with a new culture medium every day, and the culture solution cultured for 24 hours was collected to measure the secreted amount of pharmacologically active factors using ELISA. It was confirmed that the pharmacologically active factors are a vascular endothelial growth factor (VEGF), a basic fibroblast growth factor (bFGF), and matrix metalloproteinase-1 (MMP-1), which are representatively known. In each mold, 35 cell spheroids were maintained and the volume of the medium was maintained at 500 $\mu$L. The accumulative secreted amounts of pharmacologically active factors are shown in FIG. 7.

As shown in FIG. 7, it was confirmed that the expression level of the pharmacologically active factor increased as the diameter of the cell spheroid increased, and the expression level of the pharmacologically active factor was highest when the diameter was 250 $\mu$m. More specifically, it was confirmed that VEGF, which is known to induce angiogenesis, increased as the diameter increased in both AD-MSCs and BM-MSCs, and particularly in cell spheroids with a diameter of 250 $\mu$m, the expression level was increased up to 8-fold or more compared to cell spheroids with a diameter of 150 $\mu$m. In addition, it was confirmed that bFGF, which is known to promote the growth of cells, also showed the highest expression level in cell spheroids with a diameter of 250 $\mu$m. It was confirmed that in the case of MMP-1, which induces cell migration by degrading the extracellular matrix, BM-MSCs showed a low expression level, but also showed the highest expression level in cell spheroids with a diameter of 250 $\mu$m, and AD-MSCs also show the highest expression level in cell spheroids with a diameter of 250 $\mu$m. Through the above results, it was confirmed that the expression levels of pharmacologically active factors increased in cell spheroids with a diameter of 200 $\mu$m or more induced in a hypoxic environment inside cells, and through this, it was possible to predict that cell spheroids with a diameter of 200 $\mu$m to 400 $\mu$m have high tissue regeneration effects.

## Example 5: Standardization of therapeutic effects of cell spheroids

### 5.1. Confirmation of angiogenesis promoting effect of cell spheroids

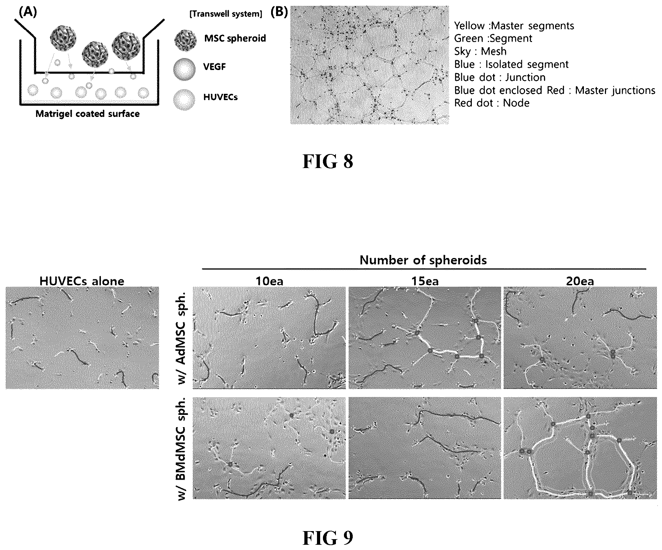

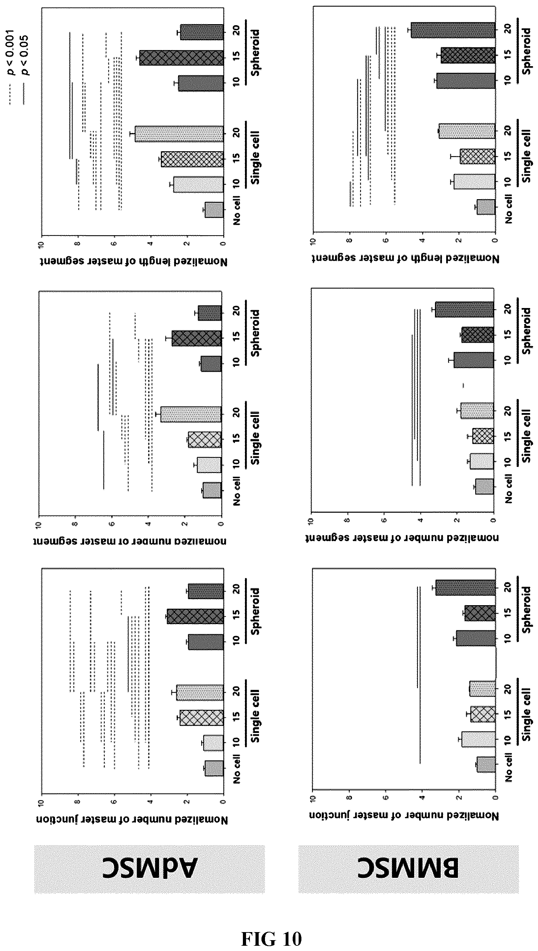

In order to confirm the effect of promoting angiogenesis of cell spheroids, tubule formation was confirmed using human umbilical vein endothelial cells (HUVECs). More specifically, in order to confirm tubule formation caused by VEGF secreted from cell spheroids, a 24-well plate was coated with a Matrigel® matrix from which growth factors had been removed, and $2.6 \times 10^4$ cells/cm$^2$ of HUVECs were seeded. Then, using a Transwell system, 10, 15, or 20 cell spheroids were each dispensed onto the top of the plate and cultured for 6 hours. As the cell spheroid, a spheroid with a diameter of 250 $\mu$m cultured on day 3 was used. After 6 hours of culture, master junctions and master segments were analyzed and quantified using ImageJ angiogenesis analysis. The experimental method is schematically shown in FIG. 8. Moreover, the experimental results are shown in FIGS. 9 and 10.

As shown in FIGS. 9 and 10, it was confirmed that when HUVECs were cultured with a BM-MSC spheroid or an AD-MSC spheroid compared to a control in which HUVECs were cultured alone, tubule formation increased, and the ability to form angiogenesis remarkably increased in 15 to 20 cell spheroids.

### 5.2. Confirmation of effect of cell spheroids on reconstructing aging vocal cord

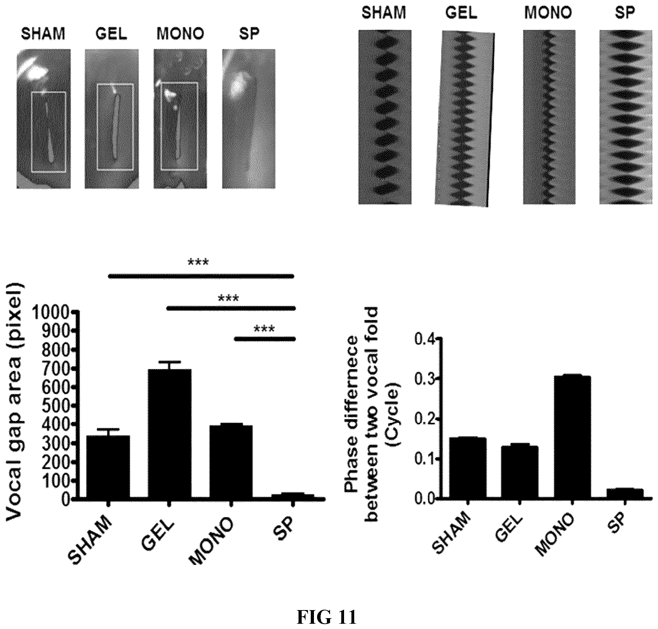

In order to confirm the aging vocal cord reconstruction effect of cell spheroids, 35 AD-MSC spheroids with a diameter of 200 $\mu$m on day 3 of culture were uniformly mixed with a hyaluronic acid hydrogel (HA gel, 1wt%), and then injected into the vocal cords of 18-month old male Sprague Dawley rats using a 25G puncture needle. As controls, a case where only HA gel was injected (GEL) and a case where the same number of single cells were injected (MONO) were prepared. Then, one month later, the degree of functional reconstruction of the vocal cord was confirmed. More specifically, in order to confirm the degree of reconstruction of vocal cord function, after a video of the movement of the vocal fold was captured using a high speed camera, a kymograph image was acquired using Meta Morph analysis software, and then the gap area that occurs when the vocal folds are closed to the maximum extent and the degree of engagement (phase difference) between both vocal cords were evaluated. Hereinafter, all animal experiments were conducted with the approval of the Institutional Animal Care and Use Committee of Seoul National University Hospital. Thereafter, all experiments were repeated at least three times, and the results were expressed as mean $\pm$ standard deviation. Statistical significance was confirmed between two groups using the Student's t-test, and among three or more groups using the Bonferroni's comparison test and then one-way ANOVA. * indicates $P < 0.05$, ** indicates $P < 0.01$, and *** indicates $P < 0.001$. The results are shown in FIG. 11.

As shown in FIG. 11, it was confirmed that in the experimental group injected with the cell spheroid (SP), the vocal cords were completely closed, whereas in the case of injecting HA gel or single cells, the gap was wide, confirming that the vocal cords were not completely closed. Furthermore, even in the results of confirming the degree of engagement of the left and right vocal cords, it was confirmed that both vocal cords matched each other during engagement only when the cell spheroid was injected. Through the above results, it could be confirmed that the cell spheroid promoted tissue regeneration to reconstruct the function of aging vocal cords.

**5.3. Confirmation of tissue restoration effect of cell spheroids**

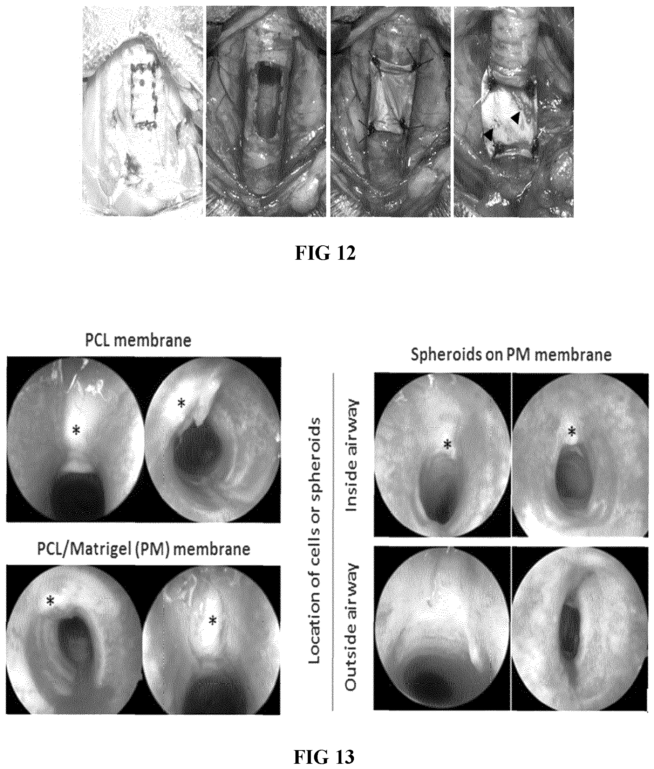

In order to confirm the tissue restoration effect of cell spheroids, a defect model was produced by creating a 5 mm × 9 mm defect in the trachea of a male New Zealand White rabbit. Then, the defective portion was sutured using a poly(caprolactone) (PCL) nanofiber membrane (9 mm × 11.5 mm), and the cell spheroid was transplanted by uniformly applying a solution in which 20 wt% of Matrigel® matrix and AD-MSC spheroids with a diameter of 200 μm were mixed at a volume ratio of 1:1 on the surface of the PCL nanofiber membrane. FIG. 12 shows an appearance in which cell spheroids are transplanted. Then, four weeks later, euthanasia was performed and an endoscopy was performed. The results are shown in FIG. 13.

As shown in FIG. 13, it was confirmed that a nanofiber membrane was confirmed with the naked eye when only the PCL nanofiber membrane was transplanted or when the PCL nanofiber membrane and a Matrigel® matrix were transplanted, but in the experimental groups where the cell spheroids were transplanted, the mucous membrane was covered with the nanofiber membrane. Through the above results, it could be confirmed that pharmacologically active factors were secreted from the transplanted stem cell spheroids to promote the regeneration of the airway lining.

Through the above results, it could be confirmed that cell spheroids with a diameter of 200 to 400 μm produced by the method of the present invention, that is, produced using the correlation function between the cell size and the diameter of the spheroid, have a hypoxic environment induced therein to promote the secretion of various pharmacologically active factors, and that this can be used for the regeneration of various tissues, blood vessels, and the like.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

[Industrial Applicability]

A standardization method for producing cell spheroids according to the present invention provides a standardization method for producing cell spheroids with remarkably increased tissue regenerative capacity, and enables the mass production of cell spheroids, which can be directly applied to treatments, and also enables the mass production of therapeutic cell spheroids, which have significantly increased tissue regenerative capacity, that is, stable quality, thus enabling industrialization of cell spheroids, and making it possible to effectively apply cell spheroids to the treatment of various diseases.

**Claims**

1. A method for producing a cell spheroid, the method comprising: a) measuring the diameter of cells;

    b) seeding $1 \times 10^3$ to $1 \times 10^{10}$ cells into a culture dish when the diameter of cells is 1 to 50 μm; and
    c) obtaining a cell spheroid by culturing the cells.

2. The method of claim 1, wherein in Step b), cells are seeded into the culture dish in an initial required number of single cells (f) calculated by the following equation:

$$f = a \cdot exp^{-0.5\left[\left(\frac{x-x_0}{b}\right)^2 + \left(\frac{y-y_0}{c}\right)^2\right]}$$

in the equation, f denotes the initial required number of single cells, x denotes the diameter of a single cell, and y denotes the diameter of a cell spheroid, and $x_0$ is 8.2775 to 10.3231, $y_0$ is 624.8576 to 726.3632, a = 53.0017 to 89.4827, b = 9.0232 to 10.2778, and c = 162.0563 to 191.5685.

3. The method of claim 1, wherein in Step c) the cells are cultured for 2 to 7 days.

**4.** The method of claim 1, wherein in Step c) the cells are cultured for 2 to 4 days.

**5.** The method of claim 1, wherein the cell spheroid has a diameter of 100 to 500 $\mu$m.

**6.** The method of claim 1, wherein the cells are somatic cells, stem cells, or a mixture thereof.

**7.** The method of claim 1, wherein the cell spheroid promotes tissue regeneration.

**8.** A cell spheroid produced by the producing method of any one of claims 1 to 7.

**9.** A cell therapy agent for promoting tissue regeneration, comprising the cell spheroid produced by the producing method of any one of claims 1 to 7 as an active ingredient.

**10.** A tissue regeneration method comprising: administering the cell spheroid produced by the producing method of any one of claims 1 to 7 to a subject in need.

**11.** A use of the cell spheroid produced by the producing method of any one of claims 1 to 7 for tissue regeneration.

**FIG 1**

**FIG 2**

**FIG 3**

| Mold | Agar mold, 81ea | | | PDMS mold, 169ea |
|---|---|---|---|---|
| Target diameter (μm) | 150 | 200 | 250 | 250 |
| Average diameter (μm) | 156.5 ± 19.4 | 201.0 ± 25.5 | 242.6 ± 29.9 | 239.8 ± 11.7 |
| Image of spheroids at day 3 of spheroidal formation | | | | |

**FIG 4**

153 ± 7.6 μm    186.3 ± 18.0 μm    255.3 ± 7.6 μm    289.5 ± 10.4 μm

312.8 ± 6.6 μm    333.3 ± 2.9 μm    359 ± 2.0 μm    383.5 ± 3.0 μm

**FIG 5**

**FIG 6**

**FIG 7**

**(A)** [Transwell system] **(B)**

MSC spheroid

VEGF

HUVECs

Matrigel coated surface

Yellow :Master segments
Green :Segment
Sky : Mesh
Blue : Isolated segment
Blue dot : Junction
Blue dot enclosed Red : Master junctions
Red dot : Node

**FIG 8**

HUVECs alone

**Number of spheroids**

10ea  15ea  20ea

w/ AdMSC sph.

w/ BMdMSC sph.

**FIG 9**

FIG 10

**FIG 11**

**FIG 12**

**FIG 13**

## INTERNATIONAL SEARCH REPORT

| International application No. |
|---|
| **PCT/KR2022/007486** |

**A.    CLASSIFICATION OF SUBJECT MATTER**

**C12N 5/071**(2010.01)i; **C12N 5/0775**(2010.01)i; **C12N 5/077**(2010.01)i; **A61K 35/28**(2006.01)i; **A61K 48/00**(2006.01)i; **A61P 17/02**(2006.01)i

According to International Patent Classification (IPC) or to both national classification and IPC

**B.    FIELDS SEARCHED**

Minimum documentation searched (classification system followed by classification symbols)

C12N 5/071(2010.01); A23L 33/10(2016.01); A61K 35/34(2006.01); A61K 47/30(2006.01); A61K 47/46(2006.01); C12N 5/074(2010.01); C12N 5/0775(2010.01); C12N 5/10(2006.01); C12Q 1/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 스페로이드(spheroid), 직경(diameter), 세포수(number of cell), 줄기세포(stem cell), 조직 재생(tissue regeneration), 분화(differentiation)

**C.    DOCUMENTS CONSIDERED TO BE RELEVANT**

| Category* | Citation of document, with indication, where appropriate, of the relevant passages | Relevant to claim No. |
|---|---|---|
| X | JP 2020-099202 A (AGC INC.) 02 July 2020 (2020-07-02)<br>   See paragraphs [0026], [0045] and [0072]-[0079]. | 1,3-9,11 |
| A | | 2 |
| A | KR 10-2019-0003871 A (EWHA UNIVERSITY - INDUSTRY COLLABORATION FOUNDATION et al.) 10 January 2019 (2019-01-10)<br>   See paragraphs [0027]-[0035] and [0117] and claims 1-12. | 1-9,11 |
| A | LAN, X. et al. Dental pulp stem cells: an attractive alternative for cell therapy in ischemic stroke. Frontiers in Neurology. 2019, vol. 10, article no. 824, inner pp. 1-10.<br>   See inner pages 2-8, and table 2. | 1-9,11 |
| A | KR 10-2019-0131853 A (KOREA INSTITUTE OF SCIENCE AND TECHNOLOGY) 27 November 2019 (2019-11-27)<br>   See claims 1-10. | 1-9,11 |

☑ Further documents are listed in the continuation of Box C.      ☑ See patent family annex.

| * Special categories of cited documents: | |
|---|---|
| "A"  document defining the general state of the art which is not considered to be of particular relevance | "T"  later document published after the international filing date or priority date and not in conflict with the application but cited to understand the principle or theory underlying the invention |
| "D"  document cited by the applicant in the international application | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| **13 September 2022** | **13 September 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/007486**

**C.    DOCUMENTS CONSIDERED TO BE RELEVANT**

| Category* | Citation of document, with indication, where appropriate, of the relevant passages | Relevant to claim No. |
|---|---|---|
| A | KR 10-2020-0047361 A (POSTECH RESEARCH AND BUSINESS DEVELOPMENT FOUNDATION) 07 May 2020 (2020-05-07)<br>See claims 1-17. | 1-9,11 |
| A | KR 10-2189850 B1 (DANKOOK UNIVERSITY CHEONAN CAMPUS INDUSTRY ACADEMIC COOPERATION FOUNDATION) 11 December 2020 (2020-12-11)<br>See claims 11, 13 and 14. | 1-9,11 |

Form PCT/ISA/210 (second sheet) (July 2019)

**INTERNATIONAL SEARCH REPORT**

| International application No. |
| --- |
| **PCT/KR2022/007486** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **10**
   because they relate to subject matter not required to be searched by this Authority, namely:

   Claim 10 pertains to a method for treatment of the human body by surgery or therapy, as well as a diagnostic method (PCT Article 17(2)(a)(i) and PCT Rule 39.(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2019)

**INTERNATIONAL SEARCH REPORT**
Information on patent family members

International application No.

**PCT/KR2022/007486**

| Patent document cited in search report | | | Publication date (day/month/year) | Patent family member(s) | | | Publication date (day/month/year) |
|---|---|---|---|---|---|---|---|
| JP | 2020-099202 | A | 02 July 2020 | WO | 2018-190305 | A1 | 18 October 2018 |
| KR | 10-2019-0003871 | A | 10 January 2019 | KR | 10-2182513 | B1 | 25 November 2020 |
| | | | | US | 2020-0131479 | A1 | 30 April 2020 |
| | | | | WO | 2019-004792 | A2 | 03 January 2019 |
| | | | | WO | 2019-004792 | A3 | 11 April 2019 |
| | | | | WO | 2019-004792 | A9 | 03 January 2019 |
| KR | 10-2019-0131853 | A | 27 November 2019 | KR | 10-2020-0019932 | A | 25 February 2020 |
| | | | | KR | 10-2264117 | B1 | 14 June 2021 |
| | | | | US | 2019-0374580 | A1 | 12 December 2019 |
| KR | 10-2020-0047361 | A | 07 May 2020 | US | 2020-0131471 | A1 | 30 April 2020 |
| KR | 10-2189850 | B1 | 11 December 2020 | KR | 10-2020-0062517 | A | 04 June 2020 |

Form PCT/ISA/210 (patent family annex) (July 2019)

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SEGERS ; VINCENT FM ; RICHARD T. LEE.** *Nature,* 2008, vol. 451 (7181), 937-942